# EUROPEAN PATENT APPLICATION

(11) **EP 1 908 516 A1**
(43) Date of publication of application: **09.04.2008**
(21) Application number: 06780741.2
(22) Date of filing: 03.07.2006
(51) Int. Cl.: B01J 20/20, A61L 9/01, A61L 9/16, B01J 20/22, B01J 20/30

(54) **ADSORBENT AND PROCESS FOR PRODUCING THE SAME**

(30) Priority: 06.07.2005 JP 2005197606; 06.07.2005 JP 2005197607
(71) Applicant: Toyo Boseki Kabushiki Kaisha, Osaka-shi, Osaka 530-8230 (JP)
(72) Inventor: HAYASHI, Toshiaki, Ohtsu-shi, Shiga 5200292 (JP)
(74) Representative: Müller-Boré & Partner Patentanwälte
(86) International application number: PCT/JP2006/313237
(87) International publication number: WO 2007/004614

(57) **Abstract**

[PROBLEMS] To provide an adsorbent that is low in the drop of lower aliphatic aldehyde adsorbing capacity over time during storage thereof, and provide a process for producing the same.

[MEANS FOR SOLVING PROBLEMS]
There is provided an adsorbent **characterized by** being composed of an active carbon of 200 µeq/g or less total surface acidic group content impregnated with aminobenzenesulfonic acid. Further, there is provided a process for producing the adsorbent.

## Description

### TECHNICAL FIELD

The present invention relates to an adsorbent used to remove a gaseous pollutant and, more particularly, to an adsorbent which has a high adsorption capacity to trap a lower aliphatic aldehyde such as acetaldehyde and is low in the drop of the adsorption capacity over time, and a process for producing the same.

### BACKGROUND ART

Bad odor and various gaseous pollutants such as a poisonous gas exist in the life space such as the inside of a room or a car. With the improvement in a living environment, it has recently become of major interest to maintain a comfortable living environment by removing these gaseous pollutants. An activated carbon has a physical adsorption capacity and is widely used to remove gaseous pollutants existing in the life space. However, the lower aliphatic aldehyde such as acetaldehyde or formaldehyde has a low boiling point and the adsorption capacity of the activated carbon to trap the lower aliphatic aldehyde is remarkably low.

To cope with this problem, there has been made a study on an adsorbent wherein an adsorption capacity to trap the lower aliphatic aldehyde is improved utilizing chemical adsorption. Since an aldehyde group has high reactivity with an amino group, amines are effective for chemical adsorption of the lower aliphatic aldehyde. Therefore, a trial of using various amines as an impregnating chemical onto a porous material has been made.

There is proposed an adsorbent wherein an activated carbon is used as the porous material, and it is impregnated with aniline, hydroxylamine hydrochloride, hydroxylamine sulfate or para-toluidine used as impregnating chemicals (Patent Documents 1 to 3). When the activated carbon is impregnated with amines, there arises a problem that added amines are oxidized by oxygen in air when stored for a long period, and thus the adsorption capacity to trap the lower aliphatic aldehyde deceases over time. Since aminobenzenesulfonic acid among amines is relatively difficult to be oxidized, an adsorbent comprising an activated carbon impregnated with aminobenzenesulfonic acid is proposed (Patent Documents 4 and 5) .

However, aminobenzenesulfonic acid is poorly soluble in a solvent such as water or alcohol and only an impregnating solution having an ultralow concentration is prepared. As a result, there is a problem that it is difficult to obtain an adsorbent having a large amount of impregnating chemicals even when the activated carbon is dipped in the impregnating solution or the impregnating solution is sprayed over the activated carbon. As a process for improving the problem, an adsorbent obtained by adding aminobenzenesulfonic acid after it was made to be soluble in water by forming into an ammonium salt, and a process for producing the same is proposed (Patent Documents 6 to 13).

Patent Document 1: Japanese Examined Patent Publication (Kokoku) No. 60-54095
Patent Document 2: Japanese Unexamined Patent Publication (Kokai) No. 56-113342
Patent Document 3: Japanese Unexamined Patent Publication (Kokai) No. 1-288336
Patent Document 4: Japanese Unexamined Patent Publication (Kokai) No. 2001-198457
Patent Document 5: Japanese Unexamined Patent Publication (Kokai) No. 2003-53179
Patent Document 6: Japanese Unexamined Patent Publication (Kokai) No. 2003-53180
Patent Document 7: Japanese Unexamined Patent Publication (Kokai) No. 2003-53181
Patent Document 8: Japanese Unexamined Patent Publication (Kokai) No. 2003-93872
Patent Document 9: Japanese Unexamined Patent Publication (Kokai) No. 2003-236374
Patent Document 10: Japanese Unexamined Patent Publication (Kokai) No. 2003-299949
Patent Document 11: Japanese Unexamined Patent Publication (Kokai) No. 2003-299951
Patent Document 12: Japanese Unexamined Patent Publication (Kokai) No. 2004-8999
Patent Document 13: Japanese Unexamined Patent Publication (Kokai) No. 2004-9000

However, even in case of the adsorbent made of activated carbon impregnated with aminobenzenesulfonic acid or ammonium aminobenzenesulfonate, a decrease in an adsorption capacity to trap a lower aliphatic aldehyde is not sufficiently suppressed when stored for a long period, and an adsorbent with less decrease in an adsorption capacity over time has been required.

### BRIEF DESCRIPTION OF THE DRAWINGS

Fig. 1 is a graph showing a change in an adsorption capacity to trap acetaldehyde, when stored for a long period, of adsorbents obtained in Examples 1A and 2A, and Comparative Examples 1A and 2A.
Fig. 2 is a graph showing a change in an adsorption capacity to trap acetaldehyde, when stored for a long period, of adsorbents obtained in Examples 1B and 2B, and Comparative Examples 1B and 2B.

### DISCLOSURE OF THE INVENTION

### PROBLEMS TO BE SOLVED BY THE INVENTION

The present invention has been made so as to solve the above problems and an objective thereof is to provide an adsorbent which has a high adsorption capacity to trap a lower aliphatic aldehyde and is low in the drop of the adsorption capacity over time, and a process for producing the same.

### MEANS FOR SOLVING THE PROBLEMS

In light of the above background art, the present invention has been made. The adsorbent and the process for producing the same, which could achieve the above objective, have constitutions as described below.

1. An adsorbent comprising an activated carbon of 200 µeq/g or less total surface acidic group content impregnated with aminobenzenesulfonic acid.

2. The adsorbent described in the paragraph 1, wherein the aminobenzenesulfonic acid is p-aminobenzenesulfonic acid,

3. A process for producing the adsorbent described in the paragraph 1 or 2, which comprises using an aqueous solution of aminobenzenesulfonic acid to impregnate an activated carbon of 200 µeq/g or less total surface acidic group content.

4. An adsorbent comprising an activated carbon of 200 *µ* eq/g or less total surface acidic group content impregnated with an ammonium aminobenzenesulfonate.

5. The adsorbent described in the paragraph 5, wherein the ammonium aminobenzenesulfonate is ammonium p-aminobenzenesulfonate.

6. A process for producing the adsorbent described in the paragraph 4 or 5, which comprises using an aqueous solution of ammonium aminobenzenesulfonate to impregnate an activated carbon of 200 µeq/g or less total surface acidic group content.

### EFFECTS OF THE INVENTION

According to the present invention, it is possible to provide an adsorbent which has a high adsorption capacity to trap a lower aliphatic aldehyde and is low in the drop of the adsorption capacity over time, and a process for producing the same.

### BEST MODE FOR CARRYING OUT THE INVENTION

The present invention will now be described in detail. It is preferred that an adsorbent of the present invention comprises activated carbon that is impregnated with aminobenzenesulfonic acid. The reasons are as follows. Namely, it is known that a primary and a secondary amine and a lower aliphatic aldehyde cause a dehydration condensation reaction to produce a Schiff's base and an enamine. Both of these reactions are equimolar reactions wherein one molecule of the amine reacts with one molecule of the lower aliphatic aldehyde. However, unlike other amines, aminobenzenesulfonic acid and the lower aliphatic aldehyde cause a Doebner-Miller reaction. In this reaction, one molecule of aminobenzenesulfonic acid reacts with two molecules of the lower aliphatic aldehyde. When amines are added onto an activated carbon, there arises a problem that the amines are oxidized by oxygen in air when stored for a long period, and thus the adsorption capacity to trap the lower aliphatic aldehyde deceases over time. Aminobenzenesulfonic acid, however, among amines is relatively difficult to be oxidized, thus preferable. Furthermore, since aminobenzenesulfonic acid has an sulfonic acid group, a lower aliphatic aldehyde and a basic gas such as ammonia can also be chemically adsorbed.

Unlike other amines, aminobenzenesulfonic acid and the lower aliphatic aldehyde cause a Doebner-Miller reaction because aminobenzenesulfonic acid is a considerably strong acidic substance. The Doebner-Miller reaction is a reaction which proceeds only in the presence of an acid catalyst and it is considered that aminobenzenesulfonic acid functions not only as a reactant, but also as an acid catalyst in this reaction.

Consequently, an impregnating chemical used in an adsorbent for the purpose of removing the lower aliphatic aldehyde is preferably aminobenzenesulfonic acid. However, even in case of the adsorbent obtained by adding aminobenzenesulfonic acid to the activated carbon, it is insufficient to suppress a decrease in an adsorption capacity to trap the lower aliphatic aldehyde when stored for a long period. Thus, it is required to develop an adsorbent which is low in the drop of the adsorption capacity over time.

Furthermore, aminobenzenesulfonic acid is poorly soluble in a solvent such as water or an alcohol. Usually, an activated carbon is impregnated with a chemical by immersing the activated carbon in an impregnating solution containing a chemical dissolved therein or spraying the impregnating solution over the activated carbon. However, only an impregnating solution having an ultralow concentration can be prepared by using aminobenzenesulfonic acid which is poorly soluble in the solvent and, as a result, it is difficult to obtain an adsorbent with large amount of impregnating chemicals.

Thus, an adsorbent obtained by adding water-solubilized ammonium aminobenzenesulfonate to an activated carbon is proposed so as to solve the above problem of aminobenzenesulfonic acid. Similarly to an aminobenzenesulfonic acid-impregnated activated carbon, an ammonium aminobenzenesulfonate-impregnated activated carbon chemically adsorbs a lower aliphatic aldehyde as a result of a reaction with it through a Doebner-Miller reaction, and is difficult to be oxidized as compared with other amines. However, even in case of the adsorbent obtained by impregnating activated carbon with ammonium aminobenzenesulfonate, it is insufficient to suppress a decrease in an adsorption capacity to trap the lower aliphatic aldehyde when stored for a long period. Thus, it is required to develop an adsorbent which is low in the drop of the adsorption capacity over time.

The present inventors have intensively studied taking notice of characteristics of an activated carbon used for supporting of an absorbent so as to suppress a decrease in an adsorption capacity to trap a lower aliphatic aldehyde over time of the adsorbent comprising an activated carbon impregnated with aminobenzenesulfonic acid and/or ammonium aminobenzenesulfonate. As the result, the inventor found that there is a correlation between the amount of total surface acidic groups of the activated carbon and the decrease in the adsorption capacity to trap the lower aliphatic aldehyde over time. Thus, the present invention has been completed.

It is important that the amount of total surface acidic groups of the activated carbon used in the present invention is 200 µeq/g or less, preferably 150 µeq/g or less, and more preferably 100 µeq/g or less. An adsorbent, which is obtained by adding aminobenzenesulfonic acid and/or ammonium aminobenzenesulfonate to an activated carbon having an amount of total surface acidic groups exceeding the above range, causes large decrease in the adsorption capacity to trap the lower aliphatic aldehyde over time. In contrast, when the amount of total surface acidic groups is too small, it become difficult to add because of poor wettability with an impregnating solution. Therefore, the amount of total surface acidic groups is preferably 0.1 µeq/g or more, and more preferably 1 µeq/g or more.

It was elucidated that oxidation of aminobenzenesulfonic acid and/or ammonium aminobenzenesulfonate, as a causative of the decrease in an adsorption capacity to trap a lower aliphatic aldehyde over time of an adsorbent comprising an activated carbon impregnated with aminobenzenesulfonic acid and/or ammonium aminobenzenesulfonate, is caused by a catalytic action of the activated carbon. Since there is a correlation between the amount of total surface acidic groups of the activated carbon and the decrease in the adsorption capacity to trap the lower aliphatic aldehyde over time and the decrease in the adsorption capacity over time is suppressed when the amount of total surface acidic groups decreases, it is considered that the catalytic activity point exists in a surface acidic group.

In the present invention, the amount of total surface acidic groups of the activated carbon was measured by the method of Boehm et al. Namely, 0.5 g of an activated carbon was placed in a flask and 50 mL of an aqueous 0.05 N sodium hydroxide solution was added, followed by standing at room temperature for 24 hours. The activated carbon and the aqueous solution in the flask were separated by filtration and the filtrate was back-titrated with an aqueous 0.05N hydrochloric acid using methyl orange as an indicator, and then the amount of total surface acidic groups was calculated.

The activated carbon usually has an amount of total surface acidic groups of 250 µeq/g or more and the amount of total surface acidic groups can be decreased by heat-treating in an inert gas atmosphere such as nitrogen.

The specific surface area of the activated carbon used in the adsorbent of the present invention is 500 m²/g or more, preferably 800 m²/g or more, and more preferably 1,000 m²/g or more. When aminobenzenesulfonic acid and/or ammonium aminobenzenesulfonate are used to impregnate the activated carbon, the adsorption capacity to trap the lower aliphatic aldehyde increases in proportion to an increase in an amount of aminobenzenesulfonic acid and/or ammonium aminobenzenesulfonate. However, when the amount of the impregnating chemical is more than a certain value (threshold value), closing of pores of the activated carbon arises and the adsorption capacity to trap the lower aliphatic aldehyde decreases as the amount of impregnating chemical increases. As the specific surface area increases, the threshold value of the amount of impregnating chemical increases. Therefore, it becomes possible to increase the adsorption capacity to trap the lower aliphatic aldehyde when an activated carbon having a large specific surface area is used. Since the activated carbon having a large specific surface area shows low yield upon production and is produced by a special method in which a carbon material is activated by adding an alkali metal, cost increases. Therefore, the specific surface area of the activated carbon used in the adsorbent of the present invention is preferably 2,500 m²/g or less, and more preferably 2,000 m²/g or less.

The amount of aminobenzenesulfonic acid and/or ammonium aminobenzenesulfonate per 1 g of the adsorbent is 0.05 mmol/g or more, preferably 0.25 mmol/g or more, and more preferably 0.5 mmol/g or more, and the upper limit is preferably 1.2 × 10⁻³ × S (mmol/g) where S (m²/g) denotes a specific surface area of the activated carbon. When the amount is less than the above range, a high adsorption capacity to trap the lower aliphatic aldehyde cannot be obtained. In contrast, when the amount is more than the above range, pores of the activated carbon are closed and it becomes impossible to effectively use the aminobenzenesulfonic acid and/or ammonium aminobenzenesulfonate for the reaction with the lower aliphatic aldehyde. Therefore, a high adsorption capacity to trap the lower aliphatic aldehyde is hardly obtained.

When a hydroxide of the alkali metal is left on the surface of the activated carbon, impregnating aminobenzenesulfonic acid is converted into an alkali metal salt and the reactivity with the lower aliphatic aldehyde is deactivated. Therefore, when pH of the activated carbon is higher than 7.5, impregnating is preferably performed after the pH was adjusted to 7.5 or lower by subjecting it to a neutralization treatment in an aqueous acid solution.

It is possible to use, as the activated carbon, granular, powdered, beads-like, and pellet form activated carbons obtained by carbonizing-activating natural products such as coconut shell, sawdust and coal and synthetic resins such as a phenol resin as raw materials; and activated carbon fibers obtained by carbonizing-activating cellulose fibers, phenol resin fibers, acryl fibers and pitch fibers. Of these activated carbons, an activated carbon derived from a phenol resin as a raw material and an activated carbon fiber derived from a phenol resin fiber as a raw material are preferred because the amount of total surface acidic groups is small.

Aminobenzenesulfonic acid includes three isomers, for example, o-aminobenzenesulfonic acid, m-aminobenzenesulfonic acid and p-aminobenzenesulfonic acid and any of these isomers can be used. Of these isomers, p-aminobenzenesulfonic acid is particularly preferred because it has strong reactivity with the lower aliphatic aldehyde.

Ammonium aminobenzenesulfonate can be obtained as a reaction product of aminobenzenesulfonic acid and ammonia. An impregnating solution of ammonium aminobenzenesulfonate can be prepared by dissolving in water a reaction product of aminobenzenesulfonic acid and ammonia, which was preliminarily obtained, or by adding aminobenzenesulfonic acid to ammonia water or by adding aminobenzenesulfonic acid and ammonium carbonate or ammonium hydrogen carbonate to water.

Ammonium aminobenzenesulfonate includes three isomers, for example, ammonium o-aminobenzenesulfonate, ammonium m-aminobenzenesulfonate and ammonium p-aminobenzenesulfonate and any of these isomers can be used. Of these isomers, ammonium p-aminobenzenesulfonate is particularly preferred because it has strong reactivity with the lower aliphatic aldehyde.

The adsorbent of the present invention is obtained by immersing an activated carbon of 200 µeq/g or less total surface acidic group content in an aqueous solution of aminobenzenesulfonic acid and/or ammonium aminobenzenesulfonate as an impregnating solution, taking out and drying the immersed activated carbon, and a method of spraying an impregnating solution over an activated carbon of 200 µeq/g or less total surface acidic group content and drying the immersed activated carbon.

The amount of aminobenzenesulfonic acid and/or ammonium aminobenzenesulfonate in the impregnating solution is from 1 to 80 parts by weight, preferably from 4 to 60 parts by weight, and more preferably from 5 to 40 parts by weight, based on 100 parts by weight of water. When the amount of aminobenzenesulfonic acid and/or ammonium aminobenzenesulfonate is below the above range, the amount of aminobenzenesulfonic acid and/or ammonium aminobenzenesulfonate to impregnate the activated carbon decreases and it becomes difficult to obtain an adsorbent having a high adsorption capacity to trap a lower aliphatic aldehyde. When using an impregnating solution in which the amount is more than the above range, the amount becomes too large, and thus pores of the activated carbon are closed and the adsorption capacity to trap the lower aliphatic aldehyde tends to decrease.

In the present invention, the amount of total surface acidic groups of the activated carbon used for being impregnated is preferably 200 µeq/g or less. The method of obtaining an activated carbon of 200 µeq/g or less total surface acidic group content includes, but is not limited to, a method of carbonizing and activating a phenol resin or a phenol resin fiber, or heat-treating an activated carbon in an inert gas atmosphere.

It is possible to use, in addition to aminobenzenesulfonic acid and/or ammonium aminobenzenesulfonate, a chemical capable of reacting with a gas component other than a lower aliphatic aldehyde such as malic acid or citric acid, and a chemical such as an antibacterial agent for imparting antibacterial properties onto the adsorbent of the present invention. However, in this case, it is necessary to select the chemical and the amount to be added so as not to drastically decrease reactivity of aminobenzenesulfonic acid and/or ammonium aminobenzenesulfonate with the lower aliphatic aldehyde. Also, the adsorbent of the present invention can be used alone as a filling layer, or mixed or used in combination with other adsorbents.

### EXAMPLES

The present invention will now be described in more detail by way of examples, but the present invention is not limited to these examples.

A test procedure of an adsorbent used in the present invention is as follows.

### <Adsorption Capacity to trap Acetaldehyde>

After weighing 1 g of an adsorbent, a glass column having an inner diameter of 12.5 mm was filled with the adsorbent. 30 ppm of an acetaldehyde-containing air adjusted to a temperature of 25°C and a relative humidity of 50% was introduced into the column at an air capacity of 5 L/min and then the concentration of acetaldehyde at a column outlet was measured every 7 minutes. The concentration of acetaldehyde was measured by a gas chromatograph equipped with FID.

A test was repeated until the concentration of acetaldehyde at a column outlet reaches 95% of the concentration at a column inlet. A value obtained by dividing an amount of acetaldehyde adsorbed on the adsorbent filled in the column during this test by the weight of the adsorbent filled was referred to as an adsorption capacity.

### Example 1A

An activated carbon having a particle size of 8 to 32 meshes, a specific surface area of 1,180 m²/g, an amount of total surface acidic groups of 291 µeq/g and pH of 6.9 was subjected to a heat treatment in a nitrogen atmosphere at 800°C for one hour. The amount of total surface acidic groups became 91 µeq/g after the heat treatment. 20 g of the above heat-treated activated carbon was placed in an impregnating solution prepared by adding 5 g of p-aminobenzenesulfonic acid to 95 g of water, followed by immersion with stirring for 3 hours. The activated carbon was taken out and then dried in a hot air dryer at 80°C for 3 hours to produce an adsorbent. The amount of a chemical on the resulting adsorbent was 0.95 mmol/g.

### Example 2A

20 g of an activated carbon fiber having a fiber size of 20 µm, a specific surface area of 1,370 m²/g, an amount of total surface acidic groups of 62 µeq/g and pH of 7.0 obtained by carbonizing-activating a phenol resin fiber was placed in an impregnating solution prepared by adding 5 g of p-aminobenzenesulfonic acid to 95 g of water, followed by immersion with stirring for 3 hours. The activated carbon fiber was taken out and then dried in a hot air dryer at 80°C for 3 hours to produce an adsorbent. The amount of a chemical on the resulting adsorbent was 1.08 mmol/g.

### Comparative Example 1A

5 g of p-aminobenzenesulfonic acid was added to 95 g of water to prepare an impregnating solution. 20 g of an activated carbon fiber used in Example 1A was placed in the impregnating solution without being subjected to a heat treatment, followed by immersion with stirring for 3 hours. The activated carbon was taken out and then dried in a hot air dryer at 80°C for 3 hours to produce an adsorbent. The amount of a chemical adsorbed on the resulting adsorbent was 0.93 mmol/g.

### Comparative Example 2A

20 g of an activated carbon fiber having a fiber size of 20 µm, a specific surface area of 1,010 m²/g, an amount of total surface acidic groups of 213 µeq/g and pH of 7.0 obtained by carbonizing-activating a pitch fiber was placed in an impregnating solution prepared by adding 5 g of p-aminobenzenesulfonic acid to 95 g of water, followed by immersion with stirring for 3 hours. The activated carbon was taken out and then dried in a hot air dryer at 80°C for 3 hours to produce an adsorbent. The amount of a chemical on the resulting adsorbent was 0.87 mmol/g.

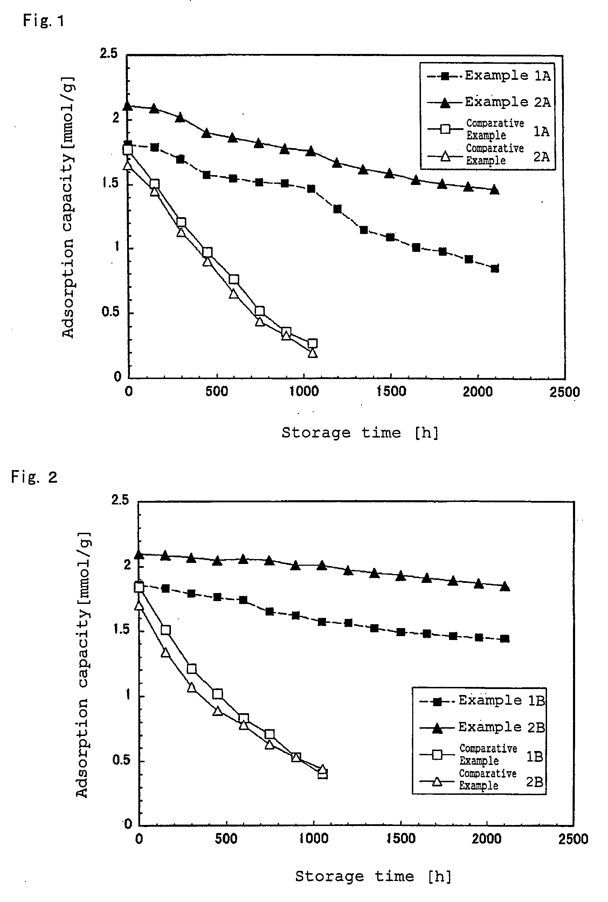

The above absorbents were stored in air at a temperature of 80°C and the adsorption capacity to trap acetaldehyde was measured by the method described above every optional storage time. A graph showing the change in an adsorption capacity to trap acetaldehyde plotted against the storage time of each adsorbent is shown in Fig. 1.

Examples 1A and 2A, wherein p-aminobenzenesulfonic acid was used to impregnate an activated carbon having a small amount of total surface acidic groups, cause less decrease in an adsorption capacity to trap acetaldehyde over time upon storage. In contrast, Comparative Examples 1A and 2A, wherein p-aminobenzenesulfonic acid was added to an activated carbon having an amount of total surface acidic groups of more than 200 µeq/g, cause large decrease in an adsorption capacity to trap acetaldehyde over time upon storage.

### Example 1B

After 25 parts by weight of p-aminobenzenesulfonic acid was added to 100 parts by weight of ammonia water having a concentration of 2.8% by weight, moisture was vaporized to obtain ammonium p-aminobenzenesulfonate as a reaction product. Also, an activated carbon having a particle size of 8 to 32 mesh, a specific surface area of 1,180 m²/g and an amount of total surface acidic groups of 291 µeq/g was subjected to a heat treatment in a nitrogen atmosphere at 800°C for one hour. The amount of total surface acidic groups became 91 µeq/g after the heat treatment. 20 g of the above heat-treated activated carbon was placed in an impregnating solution prepared by adding 10 g of ammonium p-aminobenzenesulfonate obtained above to 90 g of water, followed by immersion with stirring for one minute. The activated carbon was taken out and then dried in a hot air dryer at 80°C for 3 hours to produce an adsorbent. The amount of a chemical on the resulting adsorbent was 0.93 mmol/g.

### Example 2B

20 g of an activated carbon fiber having a fiber size of 20 µm, a specific surface area of 1,370 m²/g and an amount of total surface acidic groups of 62 µeq/g obtained by carbonizing-activating a phenol resin fiber was placed in an impregnating solution prepared by adding 10 g of ammonium p-aminobenzenesulfonate obtained in the same as in Example 1B to 90 g of water, followed by immersion with stirring for one minute. The activated carbon was taken out and then dried in a hot air dryer at 80°C for 3 hours to produce an adsorbent. The amount of a chemical on the resulting adsorbent was 1.05 mmol/g.

### Comparative Example 1B

10 g of ammonium p-aminobenzenesulfonate obtained in the same as in Example 1B was added to 90 g of water to prepare an impregnating solution. 20 g of an activated carbon used in Example 1B was placed in the impregnating solution without being subjected to a heat treatment, followed by immersion with stirring for one minute. The activated carbon was taken out and then dried in a hot air dryer at 80°C for 3 hours to produce an adsorbent. The amount of a chemical on the resulting adsorbent was 0.92 mmol/g.

### Comparative Example 2B

20 g of an activated carbon fiber having a fiber size of 20 µm, a specific surface area of 1,010 m²/g and an amount of total surface acidic groups of 213 µeq/g obtained by carbonizing-activating a pitch fiber was placed in an impregnating solution prepared by adding 10 g of ammonium p-aminobenzenesulfonate obtained in the same as in Example 1B to 90 g of water, followed by immersion with stirring for one minute. The activated carbon was taken out and then dried in a hot air dryer at 80°C for 3 hours to produce an adsorbent. The amount of a chemical on the resulting adsorbent was 0.85 mmol/g.

The above absorbents were stored in air at a temperature of 80°C and the adsorption capacity to trap acetaldehyde was measured by the method described above every optional storage time. A graph showing the change in an adsorption capacity to trap acetaldehyde plotted against the storage time of each adsorbent is shown in Fig. 2.

Examples 1B and 2B, wherein ammonium p-aminobenzenesulfonate was added onto an activated carbon having a small amount of total surface acidic groups, cause less decrease in an adsorption capacity to trap acetaldehyde over time upon storage. In contrast, Comparative Examples 1B and 2B, wherein ammonium p-aminobenzenesulfonate was added to an activated carbon having an amount of total surface acidic groups of more than 200 µeq/g, cause large decrease in an adsorption capacity to trap acetaldehyde over time upon storage.

### INDUSTRIAL APPLICABILITY

Since the present invention can provide an adsorbent which is low in the drop of an adsorption capacity to trap a lower aliphatic aldehyde over time upon storage, and a process for producing the same, the adsorbent can be utilized in wide application fields such as filters for domestic air cleaners, exhaust gas filters for business machines such as copying machine, filters for air conditioning of buildings, and automobile cabin filters. Thus, the present invention can greatly contribute to the industrial fields.

## Claims

1. An adsorbent comprising an activated carbon of 200 µeq/g or less total surface acidic group content impregnated with aminobenzenesulfonic acid.

2. The adsorbent according to claim 1, wherein the aminobenzenesulfonic acid is p-aminobenzenesulfonic acid.

3. A process for producing the adsorbent according to claim 1 or 2, comprising using an aqueous solution of aminobenzenesulfonic acid to impregnate an activated carbon of 200 µeq/g or less total surface acidic group content.

4. An adsorbent comprising an activated carbon of 200 µeq/g or less total surface acidic group content impregnated with an ammonium aminobenzenesulfonate.

5. The adsorbent according to claim 5, wherein the ammonium aminobenzenesulfonate is ammonium p-aminobenzenesulfonate.

6. A process for producing the adsorbent according to claim 4 or 5, comprising using an aqueous solution of ammonium aminobenzenesulfonate to impregnate an activated carbon of 200 µeq/g or less total surface acidic group content.
